# FASCICULE DE BREVET EUROPEEN

(11) **EP 0 929 515 B1**
(45) Date de publication et mention de la délivrance du brevet: **20.06.2001**
(21) Numéro de dépôt: 97942999.0
(22) Date de dépôt: 26.09.1997
(51) Int. Cl.: C07C 237/04, C07C 237/06, C07C 229/14, A61K 31/165

(54) **NOUVEAUX DERIVES DE LA PHENOXYETHYLAMINE, LEUR PROCEDE DE PREPARATION, LEUR APPLICATION COMME MEDICAMENTS ET LES COMPOSITIONS PHARMACEUTIQUES LES CONTENANT**
PHENOXYETHYLAMINDERIVATE, VERFAHREN ZU IHRER HERSTELLUNG, IHRE ANWENDUNG ALS ARZNEIMITTEL UND DEREN PHARMAZEUTISCHE ZUSAMMENSETZUNGEN
NOVEL PHENOXYETHYLAMINE DERIVATIVES, METHOD OF PREPARATION, APPLICATION AS MEDICINE AND PHARMACEUTICAL COMPOSITIONS CONTAINING SAME

(30) Priorité: 27.09.1996 FR 9611797
(43) Date de publication de la demande: 21.07.1999
(73) Titulaire: SOCIETE DE CONSEILS DE RECHERCHES ET D'APPLICATIONS SCIENTIFIQUES (S.C.R.A.S.), 75016 Paris (FR)
(72) Inventeur: BIGG, Denis, F-91190 Gif sur Yvette (FR); GALCERA, Marie-Odile, F-91070 Bondoufle (FR)
(74) Mandataire: Bourgouin, André
(86) Numéro de dépôt international: FR9701690
(87) Numéro de publication internationale: WO9813337

(56) Documents cités:
- WO-A-95/05366
- FR-A- 2 531 950
- SHUTSKE G M: "RECENT PATENT ACTIVITY RELATING TO SEROTONIN PHARMACOLOGY" EXPERT OPINION ON THERAPEUTIC PATENTS, vol. 4, no. 10, 1 octobre 1994, pages 1233-1241, XP000561519
- GLENNON R A: "CONCEPTS FOR THE DESIGN OF 5-HT1A SEROTONIN AGONISTS AND ANTAGONISTS" DRUG DEVELOPMENT RESEARCH, vol. 26, no. 3, 1 janvier 1992, pages 251-274, XP000562325
- FUMIHIKO OKADA ET AL.: "Antiemetic effects of serotonergic ...." THE JAPANESE JOURNAL OF PHARMACOLOGY, vol. 64, no. 2, février 1994, pages 109-114, XP000671549 cité dans la demande

## Description

Les ligands 5-HT_{1A} peuvent être utiles pour le traitement de l'anxiété, la dépression et l'hypertension (Brain 5-HT_{1A} Receptors : Behavioural and Neurochemical Pharmacology ; Editors C.T. Dourish, S. Ahlenius, P.H. Huston ; Ellis Horwood LTD, Chischester (1987)).

Il a été également démontré que les ligands 5-HT_{1A} inhibent la sécrétion de l'acide gastrique (D.C. Evans, J.S. Gidda, Gastroenterology, 104, A76 (1993)), montrent des effets anti-émétiques (F. Okada, Y. Torii, H. Saito, N. Matsuki, Jpn J. Pharmacol., 64, 109(1994)) et agissent sur la motilité du système gastrointestinal (Serotonin and Gastrointestinal Function, Editors T.S. Gaginella, J.J. Galligan ; CRC Press, Boca Raton (1995)).

La présente invention concerne de nouveaux dérivés de phénoxyéthylamine possédant une haute affinité pour le récepteur 5-HT_{1A}, des procédés pour leur préparation, des compositions pharmaceutiques les contenant et leur utilisation en tant que médicaments, notamment en tant qu'inhibiteurs de sécrétion d'acide gastrique ou qu'anti-émétiques.

Des aryloxyalkylamines et des aryloxyalkyldiamines présentant une affinité pour le récepteur 5-HT_{1A} sont décrites respectivement dans Drug Development Research (Glenonon, 26(31) 1992) et WO95/05366. Cependant ces documents ne suggèrent pas la substitution de l'amine par un groupe pentanamide.

L'invention concerne les produits de formule générale I 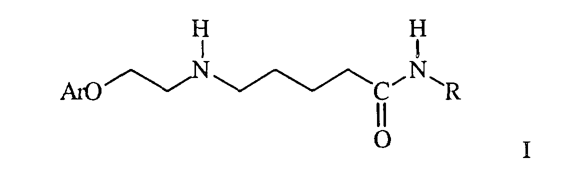 dans laquelle
- Ar: représente un phényle substitué par un ou plusieurs substituants ;
- R: représente un radical hydrocarboné contenant de 1 à 10 atomes de carbone choisi parmi les radicaux alkyle, linéaire ou ramifié, ou cycloalkyle ;
ainsi que les sels de ces produits.

L'invention a pour objet les produits de formule générale I telle que définie ci-dessus, caractérisée en ce que le ou les substituant(s) que peut porter le radical phényle que représente Ar sont choisis parmi les radicaux alkoxy inférieur, -C(O)NR₁R₂, -NHC(O)R₃, -NHC(O)NR₄R₅, -NHC(O)OR₆ dans lesquels R₁, R₂, R₃, R₄ et R₅ représentent, indépendamment, un atome d'hydrogène ou un alkyle inférieur, et R₆ représente un alkyle inférieur.

Dans les définitions indiquées ci-dessus, l'expression alkyle inférieur représente de préférence un radical alkyle ayant de 1 à 6 atomes de carbone, linéaire ou ramifié, tels que les radicaux méthyle, éthyle, propyle, isopropyle, butyle, isobutyle, sec-butyle, tert-butyle, isopentyle, néopentyle et hexyle.

Les radicaux cycloalkyles peuvent être choisis parmi les radicaux monocycliques saturés ayant de 3 à 7 atomes de carbone tels que les radicaux cyclopropyle, cyclobutyle, cyclopentyle, cyclohexyle ou cycloheptyle.

Les radicaux alkoxy inférieurs peuvent correspondre aux radicaux alkyle inférieurs indiqués ci-dessus. On préfère les radicaux méthoxy, éthoxy ou isopropyloxy.

Les produits de formule I peuvent former des sels d'addition avec les acides, en particulier les acides pharmacologiquement acceptables.

Des exemples de sels sont donnés ci-après dans la partie expérimentale.

L'invention a particulièrement pour objet les composés de formule générale I telle que décrite ci-dessus, caractérisée en ce que Ar représente un radical phényle substitué par un substituant choisi parmi les radicaux méthoxy, -C(O)NHMe, -NHC(O)Me, - NHCONH₂, -NHCONHMe et NHC(O)OMe, et en ce que R représente le radical *tert*butyle, néopentyle, cyclopentyle, cyclohexyle ou cycloheptyle.

Les substituants du radical phényle que peut représenter Ar sont de préférence situés en position 2 ou 3.

Plus particulièrement, l'invention a pour objet les produits décrits ci-après dans les exemples, en particulier les produits répondant aux formules suivantes :
- N-*tert*-butyl-5-({2-(2-méthoxyphénoxy)éthyl}amino]pentanamide ;
- N-cyclohexyl-5-[{2-(2-méthoxyphénoxy)éthyl}amino]pentanamide ;
- N-néopentyl-5-[{2-(2-méthoxyphénoxy)éthyl}amino]pentanamide ;
- N-cyclopentyl-5-[{2-(2-méthoxyphénoxy)éthyl}amino]pentanamide ;
- N-cycloheptyl-5-[{2-(2-méthoxyphénoxy)éthyl}amino]pentanamide ;
- N-cyclohexyl-5-[{2-(2-(méthylaminocarbonyl)phénoxy)éthyl}amino]pentanamide ;
- N-néopentyl-5-[{2-(2-(méthylaminocarbonyl)phénoxy)éthyl}amine]pentanamide ;
- N-néopentyl-5-[{2-(2-(aminocarbonylamino)phénoxy)éthyl}amino]pentanamide ;
- N-néopentyl-5-[{2-(3-(aminocarbonylamino)phénoxy)éthyl}amino]pentanamide ;
- N-cycloheptyl-5-[{2-(3-(aminocarbonylamino)phénoxy)éthyl}amino]pentanamide ;
- N-cyclohexyl-5-[{2-(3-(méthylcarbonylamino)phénoxy)éthyl}amino]pentanamide ;
- N-néopentyl-5-[{2-(3-(méthylcarbonylamino)phénoxy)éthyl}amino]pentanamide ;
- N-néopentyl-5-[{2-(3-(méthoxycarbonylamino)phénoxy)éthyl}amino]pentanamide ;
- N-néopentyl-5-[{2-(3-(méthylaminocarbonyl)phénoxy)éthyl}amino]butanamide,
ainsi que les sels de ces composés avec les acides minéraux ou organiques.

L'invention a également pour objet un procédé de préparation des produits de formule générale I telle que définie ci-dessus, caractérisé en ce que
A) soit l'on fait réagir un produit de formule II 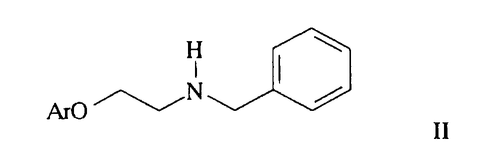 dans laquelle Ar a la signification indiquée ci-dessus, avec un produit de formule III 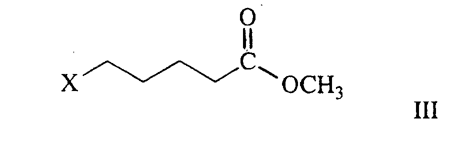 dans laquelle X représente un halogène ou un pseudo halogène, pour obtenir un produit de formule IV 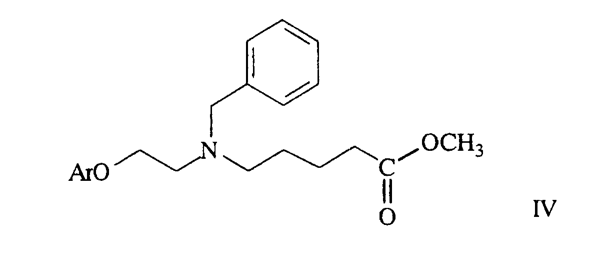 produit de formule IV que l'on traite avec une amine de formule RNH₂ dans laquelle R a la signification indiquée ci-dessus, pour obtenir un produit de formule V 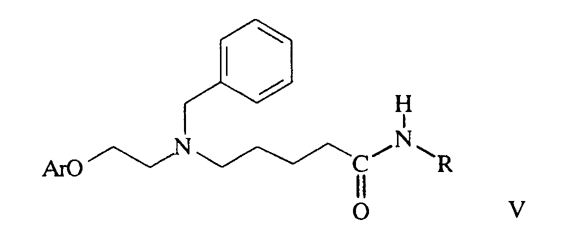 produit de formule V que l'on transforme en produit de formule I par clivage de la fonction benzyle, et produit de formule I que l'on peut transformer en sels d'acide par action de l'acide correspondant.
**B)** soit l'on fait réagir un produit de formule VI 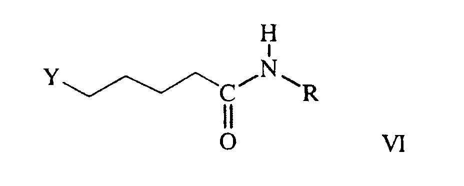 dans laquelle Y représente un radical halogène ou pseudo halogène et R a la signification indiquée ci-dessus, avec la N-benzyléthanolamine de formule 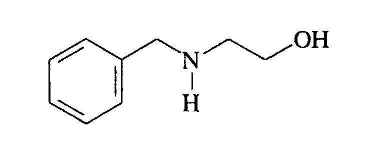 pour obtenir un produit de formule VII 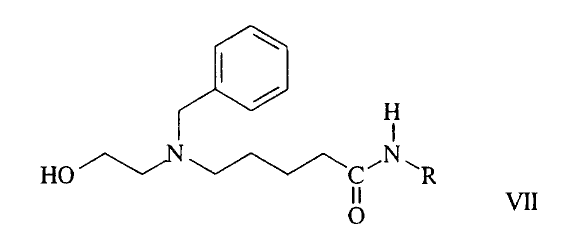 que l'on transforme en produit de formule VIII 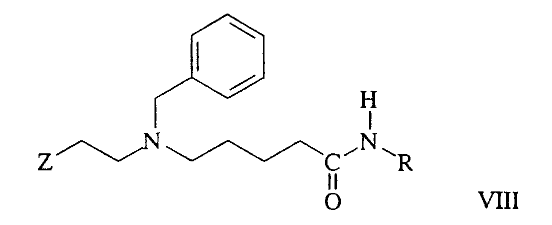
dans laquelle Z représente un radical halogène ou pseudo halogène, produit de formule VIII que l'on fait réagir avec un composé de formule générale ArOH dans laquelle Ar a la signification indiquée précédemment, pour obtenir un produit de formule V telle que définie précédemment,
produit de formule V que l'on transforme en produit de formule I par clivage de la fonction benzyle, et produit de formule I que l'on peut transformer en sels d'acide par action de l'acide correspondant.

Dans les synthèses telles que présentées ci-dessus, X, Y et Z représentent, indépendamment, un groupe partant tel que chloro, bromo, iodo, méthanesulphonyloxy, benzènesulphonyloxy ou p-toluènesulphonyloxy, en d'autres termes, un groupement halogène ou pseudo halogène.

La réaction d'un composé de formule générale II avec un composé de formule générale III pour obtenir un composé de formule générale IV peut être facilement réalisée en chauffant dans un solvant polaire, par exemple de l'acétonitrile ou du diméthylformamide, en présence d'une base inorganique telle que le carbonate de potassium ou le carbonate de sodium et éventuellement d'un catalyseur tel que l'iodure de potassium.

Les esters de formule générale IV ainsi obtenus peuvent être transformés en amides de formule générale V par réaction avec l'amine correspondante en chauffant ces deux composés, au reflux de l'amine, sans solvant, de préférence sous atmosphère d'azote ou dans un hydrocarbure aromatique en présence de tamis moléculaire.

Selon les substituants présents sur le radical phényle que représente Ar, les amides V peuvent également être obtenus après hydrolyse de la fonction ester et couplage peptidique avec les amines de formule générale RNH₂. Les amides de formule générale V peuvent également être obtenus par réaction de diéthyldiaminoaluminates de sodium ou d'un complexe amidique de lithium et d'aluminium, préparés à partir des amines de formule générale RNH₂ selon les méthodes connues comme celles décrites par exemple dans Synlett, 10, 827-8 (1994) ou dans J.Org. Chem., 57(22), 5831-4 (1992), sur les esters de formule générale IV.

Des composés de formule générale VII peuvent être préparés en chauffant un composé de formule générale VI avec de la N-benzyléthanolamine dans un solvant polaire tel qu'un alcool, en présence d'un accepteur d'acide tel qu'une amine tertiaire ou une base inorganique telle que le carbonate de sodium ou le carbonate de potassium. Alternativement, des composés de formule générale VII peuvent être aisément préparés en chauffant simplement un composé de formule générale VI avec un excès de N-benzyléthanolamine en l'absence d'un solvant, de préférence sous atmosphère d'azote et à une température comprise entre 60° C et 90° C.

Les composés de formule générale VII ainsi obtenus peuvent être transformés, par exemple, en chlorures de formule générale VIII (Z = Cl), par réaction avec du chlorure de méthanesulphonyle dans un solvant inerte, tel que le dichlorométhane, et en présence d'une base organique telle que la triéthylamine ou la diisopropyléthylamine.

Des composés de formule générale V peuvent être préparés à partir de composés de formule générale VIII en faisant réagir ces derniers avec un anion phénoxyde produit à partir du composé de formule ArOH approprié, en utilisant une base telle que l'hydroxyde de sodium, l'hydroxyde de potassium ou l'hydrure de sodium. La réaction est réalisée dans un solvant aprotique et, de préférence, dans un solvant aprotique dipolaire comme, par exemple, du diméthylformamide.

Les composés de formule générale I sont obtenus en déprotègeant les composés de formule générale V selon les méthodes générales connues de l'homme de l'art pour la débenzylation comme, par exemple, l'hydrogénation catalytique ou la réaction avec un chloroformate tel que le vinylchloroformate ou l'α-chloroéthylchloroformate suivie d'une hydrolyse ou d'une méthanolyse. D'autres méthodes de débenzylation telles que décrites dans Protective Groups in Organic Synthesis (T. W. Green, P.G.M. Wuts; 2nd Ed., J. Wiley and Sons Inc., p.364-6 (1991)) peuvent également être utilisées dans la mesure où elles sont compatibles avec les substituants sur le noyau aromatique des composés de formule générale V.

La salification éventuelle des produits de formule I est également réalisée selon les méthodes usuelles indiquées ci-après dans la partie expérimentale.

Les composés de la présente invention possèdent d'intéressantes propriétés pharmacologiques. C'est ainsi que l'on a découvert que les composés de la présente invention ont une haute affinité pour le récepteur 5HT_{1A}. Les composés de la présente invention peuvent ainsi être utilisés dans différentes applications thérapeutiques.

Les composés peuvent inhiber la sécrétion de l'acide gastrique. Ils peuvent inhiber le vomissement induit, par exemple, par le cis-platine. Ainsi, les composés de l'invention peuvent être utilisés en tant qu'anti-émétiques ou pour le traitement des maladies dans lesquelles il est nécessaire, ou souhaitable, de réduire la sécrétion de l'acide gastrique, par exemple, les ulcères gastriques ou duodénaux, les gastrites, le reflux gastroesophagial, la dyspepsie gastrique, le syndrome de Zollinger-Ellison, les nausées.

Les composés de l'invention peuvent également présenter une activité sur la vidange gastrique et la motilité intestinale. Ils peuvent ainsi être utilisés pour lutter contre la constipation, l'atonie post-chirurgicale, la gastroparésie.

Ils peuvent également être utilisés pour le traitement de certaines maladies du système nerveux telles que l'anxiété, la dépression, les troubles du sommeil tels que l'insomnie, la dépendance vis à vis de certaines drogues, la maladie d'Alzheimer, le vertige, les maladies de l'alimentation telles que l'anorexie. Les composés de l'invention peuvent également être utilisés pour traiter des maladies du système cardiovasculaire, notamment l'hypertension.

On trouvera ci-après, dans la partie expérimentale, une illustration des propriétés pharmacologiques des composés de l'invention.

Ces propriétés rendent les produits de formule I aptes à une utilisation pharmaceutique. La présente demande a également pour objet, à titre de médicaments, les produits de formule I telle que définie ci-dessus, ainsi que les sels d'addition avec les acides minéraux ou organiques pharmaceutiquement acceptables desdits produits de formule I, ainsi que les compositions pharmaceutiques contenant, à titre de principe actif, l'un au moins des médicaments tels que définis ci-dessus.

L'invention concerne ainsi des compositions pharmaceutiques contenant un composé de l'invention ou un sel additif d'acide pharmaceutiquement acceptable de celui-ci, en association avec un support pharmaceutiquement acceptable. La composition pharmaceutique peut être sous forme d'un solide, par exemple, des poudres, des granules, des comprimés, des gélules ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau, additionnés à des huiles ou des graisses pharmaceutiquement acceptables. Les compositions liquides stériles peuvent être utilisées pour les injections intramusculaires, intrapéritonéales ou sous-cutanées et les compositions stériles peuvent également être administrée par voie intraveineuse.

L'invention a également pour objet l'utilisation des produits de formule I telle que définie ci-dessus, pour la préparation de médicaments antiémétiques, de médicaments destinés à réduire la sécrétion gastrique, de médicaments destinés à accélérer la vidange gastrique, de médicaments destinés à modifier le transit intestinal, de médicaments destinés à traiter l'anxiété, la dépression, les troubles du sommeil ainsi que de médicaments destinés à traiter les maladies cardiovasculaires.

L'invention a également pour objet, à titre de produits industriels nouveaux, et notamment à titre de produits industriels nouveaux destinés à la préparation des produits de formule I, les produits de formules IV, V, VII et VIII telles que décrites ci-dessus.

Les produits de départ de l'invention, en particulier les produits de formules II, III et VI, sont des produits connus ou qui peuvent être préparés à partir de produits connus. On peut citer les références suivantes : la N-benzyléthanolamine est un produit commercialisé par exemple par la firme ACROS. Les produits de formule II peuvent être préparés par les méthodes classiques à partir des phénoxyéthylamines correspondantes par exemple par l'intermédiaire de la benzamide suivie d'une réduction par l'hydrure de lithium aluminium ou une méthode équivalente. Alternativement, on peut utiliser une amination réductrice selon les méthodes usuelles.

Les phénoxyéthylamines peuvent être préparées selon les méthodes usuelles, par exemple par réaction d'un phénol avec le chloroacétonitrile en milieu basique, réaction suivie par la réduction du nitrile par l'hydrure de lithium aluminium selon la méthode décrite dans Chim. Ther. 8(3), 259-270 (1973).

Les produits de formule III sont commerciaux ou peuvent être fabriqués par les méthodes connues de l'homme de métier. C'est ainsi que le produit de formule III, dans laquelle X représente un atome de chlore, est commercialisé par la firme ACROS.

Les produits ae formule VI peuvent etre prepares selon des methodes connues, a partir de l'acide pentanoïque, substitué en 5 par un halogène ou un pseudo-halogène, ou de dérivés activés tel que le chlorure d'acide ou l'ester activé, sur lesquels on fait réagir les amines de formule générale RNH₂.

Les dérivés phénoliques de formule générale ArOH sont commerciaux ou peuvent être fabriqués par les méthodes connues de l'homme de métier.

Les exemples suivants sont présentés pour illustrer les procédures ci-dessus et ne doivent en aucun cas être considérés comme une limite à la portée de l'invention.

### PARTIE EXPÉRIMENTALE :

### Exemple 1

### N-cyclohexyl-5-[{2-(2-méthoxyphénoxy)éthyl}amino]pentanamide (I, Ar = 2-méthoxyphényle, R = cyclohexyle : composé No. 2, Tableau 1)

### 1ère étape

### 5-[benzyl {2-(2-méthoxyphénoxy)éthyl}amino]pentanoate de méthyle (IV, Ar = 2-méthoxyphényle)

On ajoute du carbonate de potassium (28,6 g, 0,21 mole) et de l'iodure de potassium (0,2 g, 1,2 mmol) à une solution de chlorhydrate de N-benzyl[2-(2-méthoxyphénoxy)]éthylamine (26,4 g, 0,09 mole) dans le diméthylformamide (100 ml). Le mélange réactionnel est agité pendant 10 minutes à 20 °C, puis une solution de 5-chloropentanoate de méthyle (15 g, 14,3 ml, 0,1 mol) dans du diméthylformamide (30 ml) est ajoutée goutte à goutte. Le mélange est agité et chauffé à 60 °C pendant 24 heures, puis filtré et le solvant est évaporé sous pression réduite. Le résidu est repris dans du dichlorométhane (100 ml), lavé à l'eau (3 x 50 ml) et séché sur du sulfate de magnésium. La filtration et l'évaporation du solvant conduisent à l'obtention d'une huile qui est purifiée par flash-chromatographie sur du gel de silice dans un mélange acétate d'éthyle / heptane (1/1). On obtient 30 g (91 %) du composé souhaité.
RMN-¹H (CDCl₃), δ: 1,55-1,80 (m, 4H), 2,30 (t, 2H, J = 8Hz), 2,60 (t, 2H, J = 6Hz), 2,92 (t, 2H, J = 6Hz), 3,66 (s, 3H), 3,69 (s, 3H), 4,07 (t, 2H, J = 6Hz), 6,80-6,95 (m, 4H), 7,20-7,40 (m, 5H).

### 2ème étape

### N-cyclohexyl-5-[benzyl{2-(2-méthoxyphénoxy)éthyl}amino]pentanamide (V, Ar = 2-méthoxyphényle ; R=cyclohexyle)

On ajoute une solution de diéthyldihydroaluminate de sodium (4,44 ml, 8,9 mmol) à une concentration de 2 moles par litre dans le toluène, à une solution de cyclohexylamine (1,76 g, 2,03 ml, 18 mmol) dans le toluène anhydre (60 ml). Le mélange réactionnel est chauffé à 110 °C pendant 1 heure, puis une solution de 5-[benzyl{2-(2-méthoxyphénoxy)éthyl}amino]pentanoate de méthyle (6 g, 16 mmole) dans le toluène (25 ml) est ajoutée goutte à goutte. Le mélange est chauffée au reflux pendant 3 heures et laissé 18 heures à 20 °C, puis neutralisé par une solution à 10 % d'acide acétique. La phase organique est extraite puis lavée successivement par une solution saturée d'hydrogénocarbonate de sodium puis par de l'eau. Après séchage sur du sulfate de magnésium et évaporation des solvants sous pression réduite, le produit est purifié par flash-chromatographie sur du gel de silice dans un mélange acétate d'éthyle / heptane (2/1). On obtient 3,3 g (47 %) du produit souhaité, sous forme d'huile.
RMN-¹H (CDCl₃), δ : 1,00-1,80 (m, 14H), 2,10 (t, 2H, J = 6,6Hz), 2,60 (t, 2H, J = 7,6Hz), 2,90 (t, 2H, J = 6Hz), 3,67 (s, 2H), 3,85 (s, 3H), 4,08 (t, 2H, J = 6Hz), 6,80-6,90 (m, 4H), 7,25-7,40 (m, 5H).

### 3ème étape

### N-cyclohexyl-5-[{2-(2-méthoxyphénoxy)éthyl}amino]pentanamide (I, Ar = 2-méthoxyphényle, R = cyclohexyle)

On ajoute un catalyseur constitué par du palladium sur carbone humide à 10 % (1,5 g), à une solution de N-cyclohexyl-5-[benzyl {2-(2-méthoxyphénoxy) éthyl} amino] pentanamide (3 g, 6,8 mmole) dans de l'acide acétique glacial (30 ml) et le mélange est hydrogéné pendant 2 heures à 20 °C. Le catalyseur est éliminé par filtration et le solvant est évaporé sous pression réduite. Le résidu, obtenu sous forme d'un sel de l'acide acétique, est repris par du dichlorométhane (50 ml) et la base est libérée par traitement par une solution saturée d'hydrogénocarbonate de sodium. La phase organique est collectée, lavée par de l'eau, séchée sur sulfate de magnésium et le solvant est évaporé sous pression réduite. Le produit attendu est obtenu sous forme d'une poudre blanche après cristallisation dans le diéthyléther (0,95 g, 40 %).

Le traitement d'une solution de cette base libre (0,72 g) dans l'éthanol à chaud avec une solution éthanolique chaude d'acide fumarique (0,24 g) donne 0,8 g du composé No. 2 sous forme de cristaux blancs, pf = 141,5-144° C.
RMN-¹H(DMSO), δ : 1,05-1,30 (m, 5H), 1,50-1,70 (m, 9H), 2,07 (t, 2H, J = 6,6Hz), 2,94 (t, 2H, J = 6,9Hz), 3,23 (t, 2H, J = 5,3Hz), 3,50 (m, 1H), 3,77 (s, 3H), 4,21 (t, 2H, J = 5,3Hz), 6,50 (s, 2H), 6,89-7,03 (m, 4H), 7,71 (d, 1H, J = 7,8Hz).
RMN-¹³C (DMSO), δ : 22,81, 24,88, 25,31, 25,72, 32,78, 32,91, 35,07, 46,06, 47,30, 47,53, 55,53, 55,78, 65,52, 112,50, 114,74, 120,83, 121,00, 122,26, 135,02, 147,47, 149,58, 168,04, 170,88.
IR (Nujol), cm⁻¹ : 3278 (N-H), 1714 (C=O), 1635 (C=O), 1548 (C-N), 1577 (C=C), 741 (C-H aromatiques).

### Exemple 2

### N-cycloheptyl-5-[{2-(3-(aminocaibonylamino)phénoxy)éthyl}amino]pentanamide (I, Ar =3-(aminocarbonylamino)phényle, R = cycloheptyle: composé No. 10, Tableau 1)

### 1ère étape

### N-cycloheptyl-5-[benzyl(2-hydroxyéthyl)amino]pentanamide (VII, R = cycloheptyle)

On ajoute goutte à goutte du N-cycloheptyl-5-bromopentanamide (4,15 g, 15 mmol) en solution dans du diméthylformamide (20 ml) à une solution chaude (60 °C) de N-benzyléthanolamine (2,27 g, 2,13 ml, 15 mmol) dans du diméthylformamide (25 ml) en présence de carbonate de potassium (4,15 g, 3 mmol). Le mélange réactionnel est agité pendant 2 heures à 60 °C puis le solvant est évaporé sous pression réduite. Le résidu est repris dans du dichlorométhane (50 ml) et lavé par de l'eau (3x30 ml). La phase organique est collectée, séchée sur du sulfate de magnésium ; le solvant est évaporé sous pression réduite. Le produit obtenu est purifié par flash-chromatographie sur gel de silice dans un mélange dichlorométhane / méthanol (90/10) pour donner 3,04 g (50 %) du composé attendu sous forme d'huile.

### 2ème étape

### N-cycloheptyl-5-[benzyl(2-chloroéthyl)amino]pentanamide (VIII, Z = Cl, R = cycloheptyle)

On ajoute goutte à goutte du chlorure de méthanesulphonyle (0,78 g, 0,52 ml, 6,6 mmol) sous agitation à une solution refroidie de N-cycloheptyl-5-[benzyl (2-hydroxyéthyl)amino]pentanamide (2,14 g, 6 mmol) dans du dichlorométhane (20 ml) en présence de triéthylamine (0,69 g, 0,94 ml, 6,6 mmol). L'agitation est maintenue 18 heures à 20 °C. Le mélange réactionnel est lavé à l'eau glacée (25 ml) puis séché sur sulfate de magnésium. La filtration et l'évaporation du solvant sous pression réduite fournissent 2,2 g (98 %) du composé souhaité, sous forme d'huile.
RMN-¹H(CDCl₃), δ : 1,35-2,00 (m, 16H), 2,54 (t, 2H, J = 6Hz), 2,83 (t, 2H, J = 5Hz), 2,95 (t, 2H, 5Hz), 3,53 (t, 2H, J = 6Hz), 3,67 (s, 2H), 4,20 (m, 1H), 4,45 (m, 1H), 7,31 (s, 5H).

### 3ème étape

### N-cycloheptyl-5-[benzyl{2-(3-(aminocarbonylamino)phénoxy)éthyl}amino]pentanamide (V, Ar = 3-(aminocarbonylamino)phényle, R = cycloheptyle)

On ajoute du carbonate de potassium (0,92 g, 6,6 mmol) à une solution de 3-hydroxyphénylurée (1 g, 6,6 mmol) dans du diméthylformamide (20 ml) et on agite ce mélange 10 minutes à 20 °C. On ajoute ensuite goutte à goutte une solution de N-cycloheptyl-5-[benzyl-(2-chloroéthyl)amine]pentanamide (2,21 g, 6 mmol) dans du diméthylformamide (20 ml) que l'on maintient sous agitation pendant 4 heures à 80 °C. Le solvant est évaporé sous pression réduite, puis le résidu est repris par du dichlorométhane (50 ml)et lavé par de l'eau. La phase organique est récoltée et séchée puis, après évaporation du solvant, le composé souhaité est obtenu, sous forme d'huile. Il est purifié par flash-chromatographie sur gel de silice dans un mélange dichlorométhane / méthanol (95/5) pour donner 1,75 g (60 %) du composé pur.
RMN-¹H(CDCl₃), δ : 1,30-1,90 (m, 16H), 2,12 (t, 2H, J = 6Hz), 2,53 (t, 2H, J = 6Hz), 2,80 (t, 2H, J = 6Hz), 3,63 (s, 2H), 3,95 (m, 1H), 4,07 (t, 2H, J = 6Hz), 4,93 (s, 1H), 5,55 (m, 1H), 6,68 (m, 2H), 7,19-7,30 (m, 9H).

### 4ème étape

### N-cycloheptyl-5-[{2-(3-(aminocarbonylamino)phénoxy)éthyl}amine]pentanamide

On ajoute un catalyseur constitué de palladium sur carbone humide à 10 % (0,3 g), à une solution de N-cycloheptyl-5-[benzyl{2-(3-(aminocarbonylamino) phénoxy) éthyl} amino] pentanamide (0,6 g, 1,2 mmol) dans le méthanol (20 ml) et le mélange est hydrogéné pendant 24 heures à 20 °C. Ensuite, le catalyseur est filtré et remplacé par la même quantité. L'hydrogénation se poursuit à nouveau pendant 24 heures. Le catalyseur est éliminé par filtration et le solvant est évaporé sous pression réduite. Le produit souhaité est obtenu après purification par flash-chromatographie sur gel de silice dans un mélange dichlorométhane / méthanol / ammoniaque (90/10/1) (0,25 g, 51 %). Le traitement d'une solution de cette base libre (0,11 g) dans l'éthanol à chaud avec une solution éthanolique chaude d'acide fumarique (33 mg) donne 0,14 g du composé N° 10 sous forme de cristaux blancs. pf = 81-85°C.
RMN-¹H(DMSO), δ : 1,34-1,73 (m, 16H), 2,05 (t, 2H, J = 6,4Hz), 2,84 (t, 2H, J = 6,5Hz), 3,18 (t, 2H, J = 4,9Hz), 3,70 (m, 1H), 4,11 (t, 2H, J = 5,0Hz), 5,97 (s, 2H), 6,48 (s, 2H), 6,87 (d, 1H, J = 8,0Hz), 7,11 (t, 1H, J = 8,1Hz), 7,28 (m, 1H), 7,73 (d, 1H, J = 7,7Hz), 8,84 (s, 1H).
IR (KBr), cm⁻¹ : 3350 (N-H), 1700 (C=O), 1677 (urée), 1638 (C=O), 1596 (C=C), 1550 (C-N).

Les procédés décrits ci-dessus donnent un composé de l'invention sous forme d'une base-libre ou d'un sel d'addition avec un acide. Si le composé de l'invention est obtenu sous la forme d'un sel d'addition avec un acide, la base libre peut être obtenue en basifiant une solution du sel d'addition avec une base. A l'inverse, si le produit du procédé est une base libre, le sel d'addition avec un acide, en particulier un sel d'addition avec un acide pharmaceutiquement acceptable, peut être obtenu en dissolvant la base libre dans un solvant organique approprié et en traitant la solution avec un acide, selon les procédures conventionnelles de préparation des sels d'addition avec un acide à partir des bases libres.

Des exemples de sels d'addition avec un acide sont ceux dérivés d'acides inorganiques tels que les acides sulfurique, chlorhydrique, bromhydrique ou phosphorique, ou d'acides organiques tels que les acides tartrique, fumarique, maléique, citrique, caprylique, benzoïque, méthanesulphonique, p-toluènesulphonique, benzènesulphonique, succinique ou acétique.

Pour les composés de l'invention contenant un centre asymétrique, les mélanges racémiques et les isomères optiquement actifs individuels sont également considérés comme faisant partie de la portée de l'invention.

Le Tableau 1 ci-dessous montre les principaux composés préparés selon les procédures ci-dessus et qui illustrent l'invention sans en limiter la portée. Les composés Nos. 2 et 10 correspondent respectivement aux produits des exemples 1 et 2 décrits ci-dessus. Les autres produits ont été préparés en utilisant le même procédé.

**Tableau 1**

| Composé No. | Ar | R | Sel | pf (°C) |
|---|---|---|---|---|
| 1 | 2-MeO.C₆H₄ | *tert*butyle | fumarate | 146-147 |
| 2 | 2-MeO.C₆H₄ | cyclohexyle | fumarate | 141,5-144 |
| 3 | 2-MeO.C₆H₄ | néopentyle | fumarate | 128,5-131 |
| 4 | 2-MeO.C₆H₄ | cyclopentyle | fumarate | 128-129,5 |
| 5 | 2-MeO.C₆H₄ | cycloheptyle | fumarate | 131-134 |
| 6 | 2-MeNHC(O).C₆H₄ | cyclohexyle | fumarate | 126-129,5 |
| 7 | 2-MeNHC(O).C₆H₄ | néopentyle | fumarate | 143,5-145 |
| 8 | 2-H₂NC(O)NH.C₆H₄ | néopentyle | fumarate | 153-154,5 |
| 9 | 3-H₂NC(O)NH.C₆H₄ | néopentyle | fumarate | 150,5-152,5 |
| 10 | 3-H₂NC(O)NH.C₆H₄ | cycloheptyle | fumarate | 81-85 |
| 11 | 3-MeC(O)NH.C₆H₄ | cyclohexyle | hémifumarate | 176-177 |
| 12 | 3-MeC(O)NH.C₆H₄ | néopentyle | 0,75-fumarate | 165,5-168,5 |
| 13 | 3-MeOC(O)NH.C₆H₄ | néopentyle | fumarate | 124,5-125 |
| 14 | 3-MeNHC(O)NH.C₆H₄ | néopentyle | fumarate | 154,5-155,5 |

En utilisant le procédé indiqué ci-dessus, on peut également préparer les produits suivants, qui font également partie de l'invention :

| **Composé** | **Ar** | **R** |
|---|---|---|
| A | 3-MeO.C₆H₄ | éthyle |
| B | 3-EtO.C₆H₄ | néopentyle |
| C | 3-MeO.C₆H₄ | cyclohexyle |
| D | 2-NH₂C(O).C₆H₄ | méthyle |
| E | 2-NH₂C(O).C₆H₄ | cyclopentyle |
| F | 2-MeNHC(O).C₆H₄ | cyclopentyle |
| G | 3-EtNHC(O).C₆H₄ | méthyle |
| H | 2-(Me)₂NC(O).C₆H₄ | cycloheptyle |
| I | 3-EtNHC(O).C₆H₄ | cycloheptyle |
| J | 2-MeC(O)NH.C₆H₄ | néopentyle |
| K | 3-MeC(O)NH.C₆H₄ | cyclopentyle |
| L | 3-EtC(O)NH.C₆H₄ | tert-butyle |
| M | 3-HC(O)NH.C₆H₄ | cyclopentyle |
| N | 2-H₂NC(O)NH.C₆H₄ | cyclohexyle |
| O | 2-MeNHC(O)NH.C₆H₄ | néopentyle |
| P | 2-(Me)₂NC(O)NH.C₆H₄ | cycloheptyle |
| Q | 3-MeNHC(O)NH.C₆H₄ | n-propyle |
| R | 3-EtNHC(O)NH.C₆H₄ | méthyle |
| S | 2-MeOC(O)NH.C₆H₄ | néopentyle |
| T | 2-EtOC(O)NH.C₆H₄ | cycloheptyle |
| U | 3-C₃H₇OC(O)NH.C₆H₄ | *tert*-butyle |

### Etude pharmacologique des produits de l'invention

### Affinité des composés de l'invention pour le récepteur 5-HT_{1A}

L'affinité des composés pour les récepteurs 5-HT_{1A} sérotonergiques est déterminée en mesurant l'inhibition de [3H]8-hydroxy-2(di-n-propylamino)tétralin([3H]8-OH-DPAT) lié au cortex cérébral du rat, selon la méthode de Peroutka et ses collaborateurs [(J. Neurochem., 47, 529 (1986)].

Des cortex cérébraux de rats Sprague Dawley mâles sont homogénéisés dans du Tris-HCl 50 mM, pH = 7,4 et centrifugés à 40 000 g pendant 10 min à 4° C. Des pastilles sont remises en suspension dans le même tampon et incubées pendant 10 min à 37° C, et les homogénats sont de nouveau centrifugés à 40 000 g pendant 10 min à 4° C.

Des essais d'inhibition compétitive de liaison de [3H]8-OH-DPAT sont réalisés trois fois avec des compétiteurs non marqués, avec des concentrations comprises entre 100 pM et 100 µM. Des membranes de cortex cérébraux de rats (10 mg en poids humide/ ml) sont incubées avec du [3H]8-OH-DPAT (1 nM) pendant 30 min à 25° C dans du Tris-HCl 50 mM, pH = 7,4 contenant 4 mM de CaCl₂, 10 µM de pargyline et 0,1 % d'acide ascorbique.

Le [3H]8-OH-DPAT lié est séparé du [3H]8-OH-DPAT libre par filtration immédiate au moyen de filtres en fibre de verre Whatman GF/B, en utilisant un récupérateur de cellules Brandel. Les filtres sont lavés trois fois avec le même tampon à 0-4° C et leur radioactivité est étudiée au moyen d'un spectromètre à scintillation liquide.

La liaison spécifique est obtenue en soustrayant la liaison déterminée en présence de 1 µM de 8-OH-DPAT de la liaison totale. Les caractéristiques de la liaison sont analysées par analyse itérative de la régression non linéaire par ordinateur, en utilisant le programme Ligand [Munson and Rodbard, Anal. Biochem., 107, 220 (1980)].

Les résultats pour des composés représentatifs de l'invention sont donnés dans le Tableau 2 ci-après.

**Tableau 2**

| **Composé No.** | **Ki(nM)** |
|---|---|
| 1 | 0,54 |
| 2 | 0,19 |
| 3 | 0,32 |
| 4 | 0,29 |
| 5 | 0,098 |
| 9 | 0,41 |
| 10 | 0,13 |
| 11 | 0,37 |
| 12 | 0,88 |
| 13 | 0,84 |

## Revendications

1. Les composés de formule générale I: 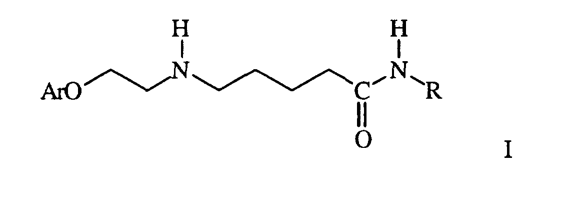 dans laquelle
Ar représente un phényle substitué par un ou plusieurs substituants choisis parmi les radicaux alkoxy inférieur, -C(O)NR₁R₂, -NHC(O)R₃, -NHC(O)NR₄R₅, -NHC(O)OR₆
dans lesquels R₁, R₂, R₃, R₄ et R₅ représentent, indépendamment, un atome d'hydrogène ou un alkyle inférieur et R₆ représente un alkyle inférieur.
R représente un radical hydrocarboné contenant de 1 à 10 atomes de carbone choisi parmi les radicaux alkyle, linéaire ou ramifié, ou cycloalkyle ;
ainsi que les sels de ces produits,
étant entendu que le terme "inférieur", lorsqu'il se réfère à un radical alkyl ou alkoxy, signifie que le radical comprend au plus 6 atomes de carbone.

2. Les composés de formule générale I telle que définie à la revendication 1, caractérisée en ce que Ar représente un radical phényle substitué par un substituant choisi parmi les radicaux méthoxy, -C(O)NHMe, -NHC(O)Me, -NHCONH₂, -NHCONHMe et NHC(O)OMe, et en ce que R représente le radical *tert*butyle, néopentyle, cyclopentyle, cyclohexyle ou cycloheptyle.

3. Les composés de formule générale I telle que définie à l'une des revendications 1 à 2 et répondant aux formules suivantes :
- N-*tert*-butyl-5-[{2-(2-méthoxyphénoxy)éthyl}amino]pentanamide ;
- N-cyclohexyl-5-[{2-(2-méthoxyphénoxy)éthyl}amino]pentanamide ;
- N-néopentyl-5-[{2-(2-méthoxyphénoxy)éthyl}amino]pentanamide ;
- N-cyclopentyl-5-[{2-(2-méthoxyphénoxy)éthyl}amino]pentanamide ;
- N-cycloheptyl-5-[{2-(2-méthoxyphénoxy)éthyl}amino]pentanamide ;
- N-cyclohexyl-5-({2-(2-(méthylaminocarbonyl)phénoxy)éthyl}amino]pentanamide;
- N-néopentyl-5-[{2-(2-(méthylaminocarbonyl)phénoxy)éthyl}amino]pentanamide;
- N-néopentyl-5-[{2-(2-(aminocarbonylamino)phénoxy)éthyl}amino]pentanamide ;
- N-néopentyl-5-[{2-(3-(aminocarbonylamino)phénoxy)éthyl}amino]pentanamide ;
- N-cycloheptyl-5-[{2-(3-(aminocarbonylamino)phénoxy)éthyl}amino]pentanamide ;
- N-cyclohexyl-5-[{2-(3-(méthylcarbonylamino)phénoxy)éthyl}amino]pentanamide ;
- N-néopentyl-5-[{2-(3-(méthylcarbonylamino)phénoxy)éthyl}amino]pentanamide ;
- N-néopentyl-5-[{2-(3-(méthoxycarbonylamino)phénoxy)éthyl}amino]pentanamide ;
- N-néopentyl-5-[{2-(3-(méthylaminocarbonyl)phénoxy)éthyl}amino]pentanamide,
ainsi que les sels de ces composés avec les acides minéraux ou organiques.

4. Procédé de préparation des produits de formule générale I telle que définie à la revendication 1, caractérisé en ce que
**A)** soit l'on fait réagir un produit de formule II 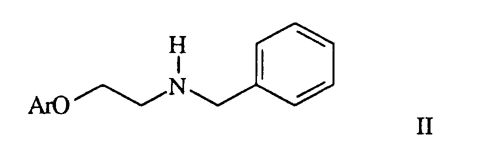 dans laquelle Ar a la signification indiquée à la revendication 1, avec un produit de formule III 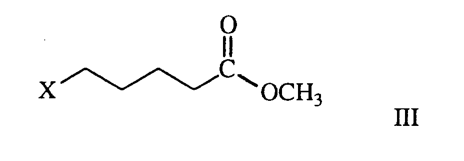 dans laquelle X représente un halogène ou un pseudo halogène, pour obtenir un produit de formule IV 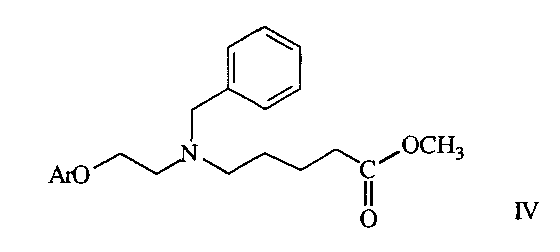 produit de formule IV que l'on traite avec une amine de formule RNH₂ dans laquelle R a la signification indiquée à la revendication 1, pour obtenir un produit de formule V 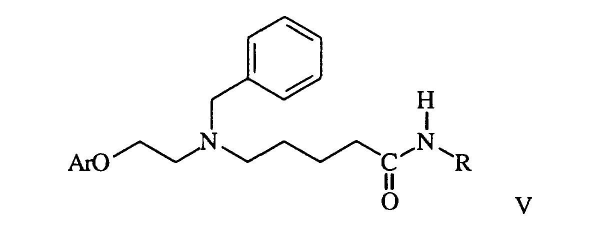
**B)** soit l'on fait réagir un produit de formule VI 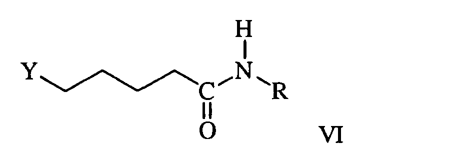 dans laquelle Y représente un radical halogène ou pseudo halogène et R a la signification indiquée à la revendication 1, avec la N-benzyléthanolamine de formule 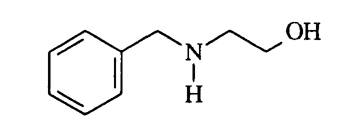 pour obtenir un produit de formule VII 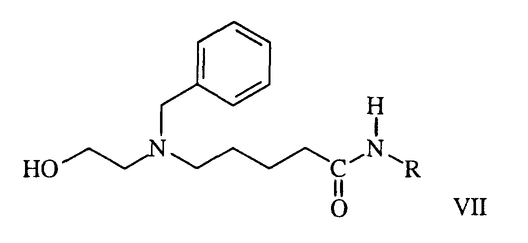 que l'on transforme en produit de formule VIII 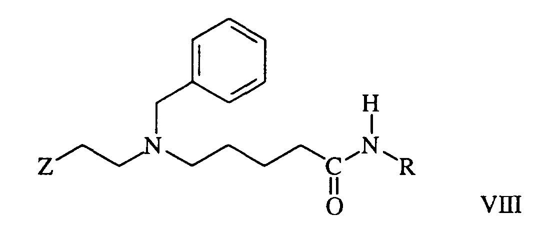 dans laquelle Z représente un radical halogène ou pseudo halogène, produit de formule VIII que l'on fait réagir avec un composé de formule générale ArOH dans laquelle Ar a la signification indiquée à la revendication 1, pour obtenir un produit de formule V telle que définie précédemment,
produit de formule V que l'on transforme en produit de formule I par clivage de la fonction benzyle, et produit de formule I que l'on peut transformer en sels d'acide par action de l'acide correspondant.

5. A titre de médicaments, les produits de formule I telle que définie à la revendication 1, ainsi que les sels d'addition avec les acides minéraux ou organiques pharmaceutiquement acceptables desdits produits de formule I.

6. A titre de médicaments, les produits de formule I telle que définie à l'une des revendications 2 à 3, ainsi que les sels d'addition avec les acides minéraux ou organiques pharmaceutiquement acceptables desdits produits de formule I.

7. Compositions pharmaceutiques contenant, à titre de principe actif, l'un au moins des médicaments tels que définis à l'une des revendications 5 ou 6.

8. Utilisation des produits de formule I telle que définie à l'une quelconque des revendications 1 à 3 pour la préparation de médicaments antiémétiques.

9. Utilisation des produits de formule I telle que définie à l'une quelconque des revendications 1 à 3 pour la préparation de médicaments destinés à réduire la sécrétion gastrique.

10. Utilisation des produits de formule I telle que définie à l'une quelconque des revendications 1 à 3 pour la préparation de médicaments destinés à accélérer la vidange gastrique.

11. Utilisation des produits de formule I telle que définie à l'une quelconque des revendications 1 à 3 pour la préparation de médicaments destinés à modifier le transit intestinal.

12. Utilisation des produits de formule I telle que définie à l'une quelconque des revendications 1 à 3 pour la préparation de médicaments destinés à traiter l'anxiété, la dépression, les troubles du sommeil.

13. Utilisation des produits de formule I telle que définie à l'une quelconque des revendications 1 à 3 pour la préparation de médicaments destinés à traiter les maladies cardiovasculaires, notamment l'hypertension.

14. A titre de produits industriels nouveaux, les composés de formules IV, V, VII et VIII telles que définies à la revendication 4.

## Patentansprüche

1. Verbindungen der allgemeinen Formel I: 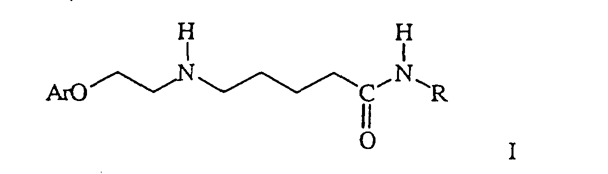 in der
Ar ein Phenyl darstellt, das durch einen oder mehrere Substituenten substituiert ist, die ausgewählt sind aus den Resten: niederer Alkoxyrest, -C(O)NR₁R₂, -NHC(O)R₃, -NHC(O)NR₄R₅, -NHC(O)OR₆,
in denen R₁, R₂, R₃, R₄ und R₅ voneinander unabhängig ein Wasserstoffatom oder einen niederen Alkylrest und R₆ einen niederen Alkylrest darstellen,
R einen Kohlenwasserstoffrest mit 1 bis 10 Kohlenstoffatomen darstellt, der unter verzweigten oder unverzweigten Alkylresten oder Cycloalkylresten ausgewählt ist,
sowie die Salze dieser Produkte,
wobei der Begriff "niederer" im Zusammenhang mit einem Alkyl- oder Alkoxyrest bedeutet, dass der Rest höchstens 6 Kohlenstoffatome enthält.

2. Verbindungen der in Anspruch 1 definierten allgemeinen Formel I, dadurch gekennzeichnet, dass Ar einen Phenylrest darstellt, der durch einen Substituent substituiert ist, der aus folgenden Resten ausgewählt ist: Methoxyrest, -C(O)NHMe, -NHC(O)Me, -NHCONH₂, -NHCONHMe und NHC(O)OMe, und dass R den *tert*.-Butyl-, Neopentyl-, Cyclopentyl-, Cyclohexyl- oder Cycloheptylrest darstellt.

3. Verbindungen der in einem der Ansprüche 1 bis 2 definierten allgemeinen Formel I, die den folgenden Formeln entsprechen:
- N- *tert*,-Butyl-5-[{2- (2-methoxyphenoxy)ethyl}amino]pentanamid,
- N-Cyclohexyl-5-[{2-(2-methoxyphenoxy)ethyl}amino]pentanamid,
- N-Neopentyl-5-[{2-(2-methoxyphenoxy)ethyl}amino]pentanamid,
- N-Cyclopentyl-5-[{2-(2-methoxyphenoxy)ethyl}amino]pentanamid,
- N-Cycloheptyl-5-[{2-(2-methoxyphenoxy)ethyl}amino]pentanamid,
- N-Cyclohexyl-5-[{2-(2-(methylaminocarbonyl)phenoxy)ethyl} amino]pentanamid,
- N-Neopentyl-5-[{2-(2-(methylaminocarbonyl)phenoxy)ethyl} amino] pentanamid,
- N-Neopentyl-5-[{2-(2-(aminocarbonylamino)phenoxy)ethyl} amino]pentanamid,
- N-Neopentyl-5-[{2(3-(aminocarbonylamino)phenoxy)ethyl} amino] pentanamid,
- N-Cycloheptyl-5-[{2-(3-(aminocarbonylamino)phenoxy)ethyl} amino] pentanamid,
- N-Cyclohexyl-5-[{2-(3-(methylcarbonylamino)phenoxy)ethyl) amino] pentanamid,
- N-Neopentyl-5-[{2-(3-(methylcarbonylamino)phenoxy)ethyl} amino]pentanamid,
- N-Neopentyl-5-[{2-(3-(methoxycarbonylamino)phenoxy)ethyl} amino]pentanamid,
- N-Neopentyl-5-[{2-(3-(methylaminocarbonyl)phenoxy)ethyl} amino]pentanamid,
sowie die Salze dieser Verbindungen mit den Mineralsäuren oder organischen Säuren.

4. Verfahren zur Herstellung der Produkte der allgemeinen Formel I gemäß Anspruch 1, dadurch gekennzeichnet, dass man
A) entweder ein Produkt der Formel II 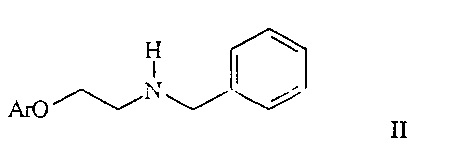 in der Ar die in Anspruch 1 angegebene Bedeutung hat, mit einem Produkt der Formel III 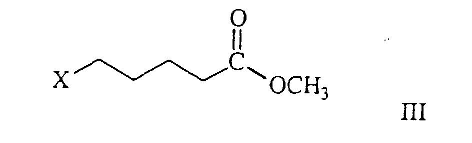 in der X ein Halogen oder ein Pseudohalogen bedeutet, reagieren lässt, um ein Produkt der Formel IV 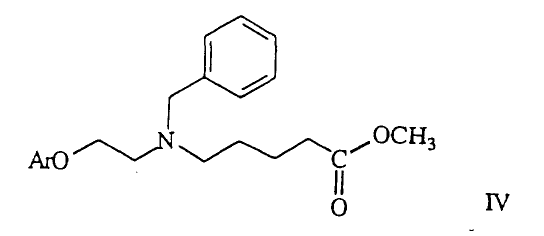 zu erhalten, das man mit einem Amin der Formel RNH₂ behandelt, in der R die in Anspruch 1 angegebene Bedeutung hat, um ein Produkt der Formel V 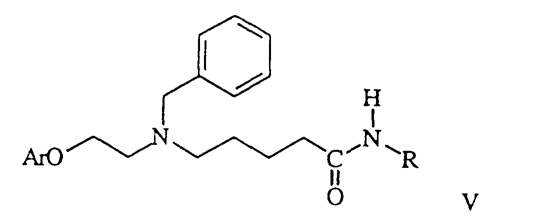 zu erhalten,
B) oder ein Produkt der Formel VI 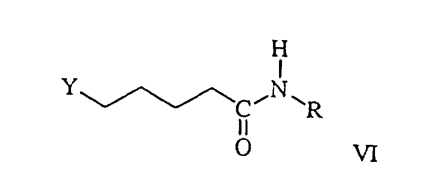 in der Y einen Halogen- oder Pseudohalogenrest bedeutet und R die in Anspruch 1 angegebene Bedeutung hat, mit N-Benzylethanolamin der Formel 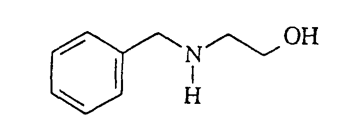 reagieren lässt, um ein Produkt der Formel VII 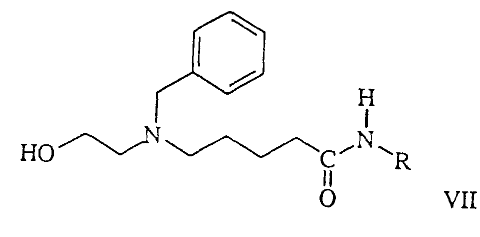 zu erhalten, das man in ein Produkt der Formel VIII 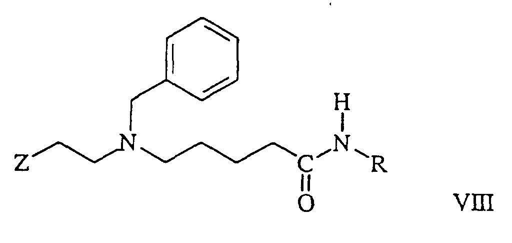 umwandelt, in der Z einen Halogen- oder Pseudohalogenrest darstellt, wobei man dieses Produkt der Formel VIII mit einer Verbindung der allgemeinen Formel ArOH, in der Ar die in Anspruch 1 angegebene Bedeutung hat, reagieren lässt, um ein Produkt der oben definierten Formel V zu erhalten,
das man durch Abspalten der Benzyl-Funktion in ein Produkt der Formel I umwandelt, wobei man das Produkt der Formel I durch Umsetzung mit der entsprechenden Säure in Säuresalze umwandeln kann.

5. Die Produkte der in Anspruch 1 definierten Formel I sowie die mit den pharmazeutisch verträglichen mineralischen und organischen Säuren gebildeten Additionssalze dieser Produkte der Formel I in Form von Arzneimitteln.

6. Die Produkte der in einem der Ansprüche 2 bis 3 definierten Formel I sowie die mit den pharmazeutisch verträglichen Mineralsäuren oder organischen Säuren gebildeten Additionssalze dieser Produkte der Formel I in Form von Arzneimitteln.

7. Pharmazeutische Zusammensetzungen, die als Wirkstoff mindestens eines der Arzneimittel nach einem der Ansprüche 5 oder 6 enthalten.

8. Verwendung der Produkte der in einem der Ansprüche 1 bis 3 definierten Formel I für die Herstellung von Antiemetika.

9. Verwendung der Produkte der in einem der Ansprüche 1 bis 3 definierten Formel I für die Herstellung von Arzneimitteln zur Verringerung der Magensekretion.

10. Verwendung der Produkte der in einem der Ansprüche 1 bis 3 definierten Formel I für die Herstellung von Arzneimitteln zur Beschleunigung der Magenentleerung.

11. Verwendung der Produkte der in einem der Ansprüche 1 bis 3 definierten Formel I für die Herstellung von Arzneimitteln zur Modifizierung des Darmdurchgangs.

12. Verwendung der Produkte der in einem der Ansprüche 1 bis 3 definierten Formel I für die Herstellung von Arzneimitteln zur Behandlung von Angstzuständen, Depressionen und Schlafstörungen.

13. Verwendung der Produkte der in einem der Ansprüche 1 bis 3 definierten Formel I für die Herstellung von Arzneimitteln zur Behandlung von kardiovaskulären Erkrankungen, insbesondere Hypertonie.

14. Die Verbindungen der Formeln IV, V, VII und VIII nach Anspruch 4 in Form von neuen Industrieprodukten.

## Claims

1. The compounds of general formula I: 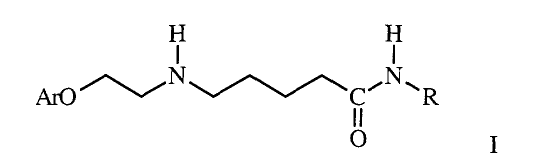 in which
Ar represents a phenyl substituted by one or more substitents chosen from the lower alkoxy, -C(O)NR₁R₂, -NHC(O)R₃, -NBC(O)NR₄R₅, -NHC(O)OR₆ radicals
in which R₁, R₂, R₃, R₄ and R₅ represent, independently, a hydrogen atom or a lower alkyl and R₆ represents a lower alkyl ;
R represents a hydrocarbon radical containing 1 to 10 carbon atoms chosen from linear or branched alkyl, or cycloalkyl radicals ;
and salts of said products,
it being understood that the term "alkyl", when it refers to an alkyl or an alkoxy radical, means that the radical contains no more than 6 carbon atoms.

2. The compounds of general formula I as defined in claim 1, characterised in that Ar represents a phenyl radical substituted by a substituent chosen from the methoxy, -C(O)NHMe, -NHC(O)Me, -NHCONH₂, -NHCONHMe and NHC(O)OMe radicals, and in that R represents the *tert*-butyl, neopentyl, cyclopentyl, cyclohexyl or cycloheptyl radical.

3. The compounds of general formula I as defined in one of claims 1 to 2 and corresponding to the following formulae :
- N-*tert*-butyl-5-[{2-(2-methoxyphenoxy)ethyl}amino]pentanamide;
- N-cyclohexyl-5-[{2-(2-methoxyphenoxy)ethyl}amino]pentanamide;
- N-neopentyl-5-[{2-(2-methoxyphenoxy)ethyl}amino]pentanamide;
- N-cyclopentyl-5-[{2-(2-methoxyphenoxy)ethyl}amino]pentanamide ;
- N-cycloheptyl-5-[{2-(2-methoxyphenoxy)ethyl}amino]pentanamide;
- N-cyclohexyl-5- [ {2-(2-(methylaminocarbonyl)phenoxy)ethyl}amino]pentanamide ;
- N-neopentyl-5-[{2-(2-(methylaminocarbonyl)phenoxy)ethyl}amino]pentanamide;
- N-neopentyl-5-[{2-(2-(aminocarbonylamino)phenoxy)ethyl}amino]pentanamide;
- N-neopentyl-5-[{2-(3-(aminocarbonylamino)phenoxy)ethyl}amino]pentanamide;
- N-cycloheptyl-5-[{2-(3-(aminocarbonylamino)phenoxy)ethyl}amino]pentanamide;
- N-cyclohexyl-5-[{2-(3-(methylcarbonylamino)phenoxy)ethyl]}amino]pentanamide;
- N-neopentyl-5-[{2-(3-(methylcarbonylamino)phenoxy)ethyl}amino]pentanamide;
- N-neopentyl-5-[{2-(3-(methoxycarbonylamino)phenoxy)ethyl}amino]pentanamide;
- N-neopentyl-5-[{2-(3-(methylaminocarbonyl)phenoxy)ethyl}amino]pentanamide,
and the salts of said compounds with inorganic or organic acids.

4. A process for the preparation of products of general formula I as defined in claim 1, characterised in that
**A)** either a product of formula II 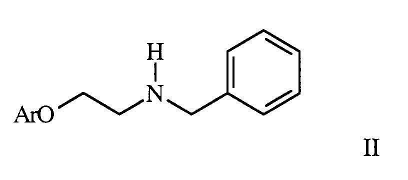 in which Ar has the meaning given in claim 1, is allowed to react with a product of formula III 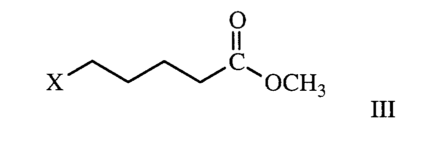 in which X represents a halogen or a pseudo halogen, in order to obtain a product of formula IV 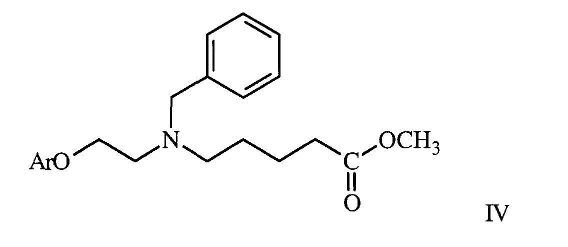 product of formula IV which is treated with an amine of formula RNH₂ in which R has the meaning given in claim 1, in order to obtain a product of formula V 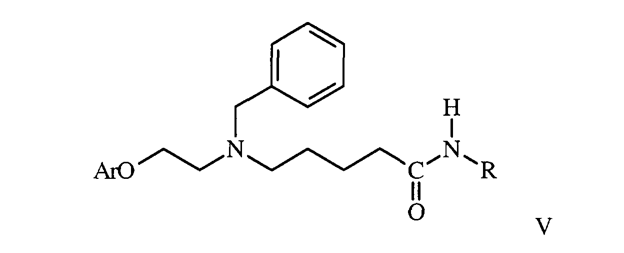
**B)** or a product of formula VI 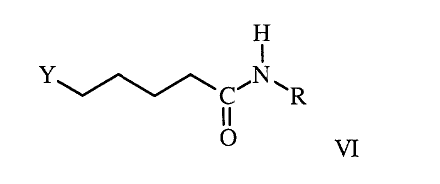 in which Y represents a halogen or pseudo halogen radical and R has the meaning given in claim 1, is allowed to react with N-benzylethanolamine of formula 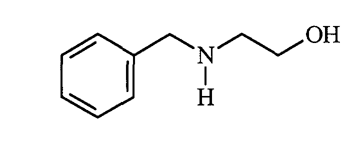 in order to obtain a product of formula VII 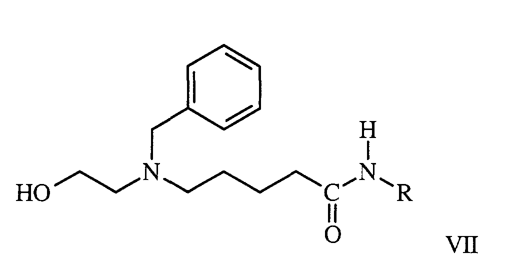 which is converted to a product of formula VIII 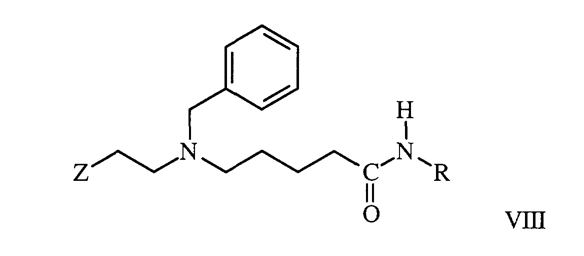 in which Z represents a halogen or pseudo halogen radical, product of formula VIII that is allowed to react with a compound of general formula ArOH in which Ar has the meaning given in claim 1, in order to obtain a product of formula V as defined above, product of formula V that is converted to a product of formula I by cleavage of the benzyl function, and product of formula I that may be converted to acid salts by the action of the corresponding acid.

5. As medicaments, the products of formula I as defined in claim 1, and the addition salts with pharmaceutically acceptable inorganic or organic acids of the said products of formula I.

6. As medicaments, the products of formula I as defined in one of claims 2 to 3, and the addition salts with pharmaceutically acceptable inorganic or organic acids of the said products of formula I.

7. Pharmaceutical compositions containing, as active principle, at least one of the medicaments as defined in one of claims 5 or 6.

8. Use of the products of formula I as defined in any one of claims 1 to 3 for the preparation of anti-emetic medicaments.

9. Use of the products of formula I as defined in any one of claims 1 to 3 for the preparation of medicaments intended to reduce gastric secretion.

10. Use of the products of formula I as defined in any one of claims 1 to 3 for the preparation of medicaments intended to accelerate gastric emptying.

11. Use of the products of formula I as defined in any one of claims 1 to 3 for the preparation of medicaments intended to modify intestinal transit.

12. Use of the products of formula I as defined in any one of claims 1 to 3 for the preparation of medicaments intended to treat anxiety, depression, sleep disorders.

13. Use of the products of formula I as defined in any one of claims 1 to 3 for the preparation of medicaments intended to treat cardiovascular diseases, particularly hypertension.

14. As new industrial products, the compounds of formulae IV, V, VII and VIII as defined in claim 4.
